# EUROPÄISCHE PATENTANMELDUNG

(11) **EP 2 465 463 A1**
(43) Veröffentlichungstag der Anmeldung: **20.06.2012**
(21) Anmeldenummer: 10195969.0
(22) Anmeldetag: 20.12.2010
(51) Int. Cl.: A61C 13/00, B24B 19/22, B24B 51/00

(54) **Verfahren und Vorrichtung zur extraoralen Zahnersatzherstellung**

(71) Anmelder: Weyrauch, Jens, 63820 Elsenfeld (DE)
(72) Erfinder: Weyrauch, Jens, 63820 Elsenfeld (DE)
(74) Vertreter: Zimmermann & Partner

(57) **Zusammenfassung**

Die Erfindung betrifft ein Verfahren und eine Vorrichtung zur extraoralen Herstellung eines Zahnersatzteiles, wobei das Verfahren Zur Verfügung Stellen eines Basiskörpers (300); Drehschleifen des Basiskörpers; und Schleifen des Basiskörpers umfasst. Des Weiteren ist ein Frässchritt möglich. Das vorgestellte Verfahren reduziert die Herstellungszeit deutlich, da sie die benötigte Zeit zum Fräsen minimiert.

## Beschreibung

### Technisches Gebiet

Die Erfindung betrifft ein Verfahren und eine Vorrichtung zur Herstellung eines Zahnersatzes. Die Erfindung betrifft insbesondere ein Verfahren zur Herstellung eines extraoral gefertigten Zahnersatzes, der durch Zerspanen aus einem Basiskörper, insbesondere einem stabförmigen Basiskörper hergestellt wurde.

### Stand der Technik

In der Zahnheilkunde existieren im Wesentlichen zwei Verfahren zur Restauration von Zähnen mit Hartsubstanzdefekten, die zum Beispiel durch Karies hervorgerufen sind. Die Defekte werden entweder mit plastischem Füllungsmaterial direkt am Zahn behandelt, indem das defekte Zahnmaterial durch Bohren entfernt wird, und anschließend mit einem flüssigen bzw. viskosen Füllmaterial befüllt wird. Nach Aushärtung und Nachbearbeitung des Füllungsmaterials ist keine weitere therapeutische Maßnahme mehr erforderlich.

Die vorliegende Erfindung betrifft hingegen extraoral gefertigten Zahnersatz. Hierzu zählen kleine Keramikaufbauten, Inlays, Onlays, Veneers und Kronen. Hierzu zählen nicht Befestigungsmittel, die zur Verankerung im Knochen ausgebildet sind, wie zum Beispiel Implantate. Zum Ersatz ganzer Zähne dienen Brücken, deren Pfeilerzähne wie zur Aufnahme von Kronen präpariert werden. Bei den extraoral gefertigten Therapiemittel findet die Herstellung der Therapiemittel außerhalb des Mundraums statt, wobei die zur Herstellung nötigen Informationen im Mundraum konventionell mit Abformmassen erfasst oder mit Scannern intraoral vermessen wurden. Basierend auf diesen Vorgaben, die gegebenenfalls noch durch weitere Informationen vom Zahnarzt ergänzt werden, werden die Therapiemittel hergestellt. Die fertig gestellten Therapiemittel werden wiederum vom Zahnarzt auf den präparierten Zähnen bspw. mit einem speziellen Zement wie Zink-Phosphat-Zement befestigt.

Im Stand der Technik wird der Zahnersatz beispielsweise gegossen. Dazu wird der vom Zahnarzt genommene Abdruck oder ein auf dieser Form basierender Formkörper mit flüssigem Material, wie zum Beispiel Gold, ausgegossen. Weitere Vorgaben, zum Beispiel über die gewünschte Kauflächenstruktur, werden regelmäßig vom behandelnden Zahnarzt an den für die Zahnersatzteilherstellung zuständigen Zahntechniker gegeben. Zahlreiche Werkstoffe für Zahnersatzteile sind Metalle, Keramiken, oder Komposite. Sie haben regelmäßig einen über 1.000°C liegenden Schmelzpunkt.

Die vorliegende Anmeldung betrifft Zahnersatzteile, die durch mechanische Zerspanverfahren hergestellt werden. Dabei soll Zerspanen alle mechanischen Bearbeitungsverfahren beinhalten, bei denen das Material in die gewünschte Form gebracht wird, indem überflüssiges Material in Form von Spänen abgetragen wird.

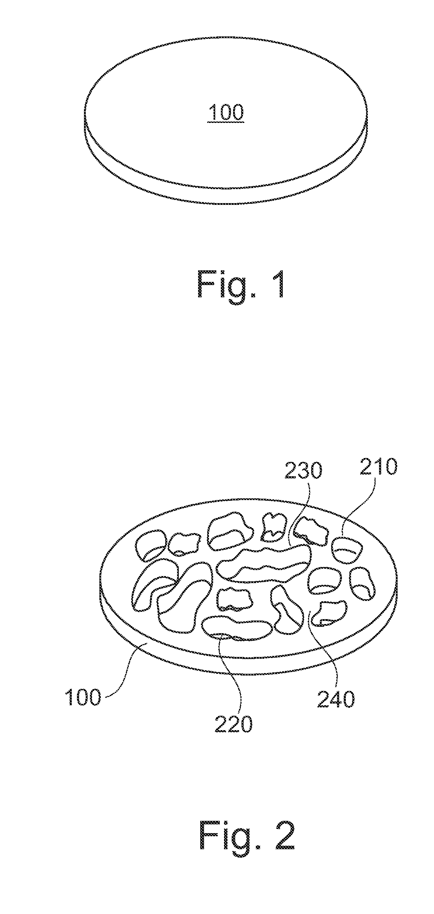

Im Stand der Technik erfolgt die Herstellung von daher durch Fräsen aus Platten geeigneter Höhe. Dies wird in Fig. 1 veranschaulicht. Aus einer Platte 100 von z. B. 10 mm Höhe und einem Durchmesser von z. B. 10 cm wird mit Hilfe von Fräsern eine Anzahl von ca. 10-30 Zahnersatzteilen herausgefräst. Eine solche Platte vor der Fräsbearbeitung ist in Fig. 1 gezeigt. In Anbetracht der zu fräsenden Teile wird eine möglichst sinnvolle Plattenaufteilung gewählt, wobei dann im Frässchritt die einzelnen Zahnersatzteile herausgefräst werden. Wie in Fig. 2 veranschaulicht, gibt es dabei beispielsweise kleinere Zahnersatzteile, die damit auch zu kleineren Fräslöchern 210 führen, sowie größere Zahnersatzteile, weil sie bspw. mehrere Zähne verbindende Brücken betreffen, die damit auch zu größeren Fräslöchern 220 bzw. 230 führen. Dabei ist es Ziel, die Fräsungen so anzuordnen, dass die Menge des in den Platten verbleibenden Materials 240 so gering wie möglich ist.

Beim Fräsen wird mit Hilfe von einem rotierenden Präzisions-Schneidewerkzeug mit spitzem Fräskopf Material aus der Platte entfernt, bis schließlich das Zahnersatzteil geformt ist. Zusätzlich werden manchmal mindestens zwei Anker zur Verbindung zwischen Zahnersatzteil und Platte übrig gelassen, die eine dünne Befestigung der Zahnersatzteile an der Platte darstellen und damit ein Verbleiben des Zahnersatzteils in der Platte garantieren. So wird zum Beispiel eine Platte mit sämtlichen ausgefrästen, aber noch in der Platte über die Anker fixierten Zahnersatzteile als Ganzes an ein Zahnlabor oder einen Zahnarzt geliefert, der dann lediglich - ebenfalls mit einem Fräswerkzeug - die Zahnersatzteile aus der Platte lösen muss und dem Patienten einsetzen muss.

Allerdings ist dieses Verfahren zeitaufwändig und somit kostenintensiv. Ein typischer Zeitaufwand pro Zahnersatzteil liegt bei etwa 15 Minuten. Die optimale Materialausnutzung wird nur selten erreicht, und dennoch fließt viel Zeit darin ein, die Fräslöcher so anzusetzen, dass möglichst wenig überflüssiges Material in den Platten verbleibt. Darüber hinaus sind manche Zahnersatzteile derart zu strukturieren, dass nicht alle Stellen gut von oberhalb oder unterhalb der Platte zugänglich sind. Das führt schließlich zu relativ großen Fräslöchern, wobei ein nicht unbeachtlicher Teil dieser Fräslöcher lediglich dafür gefräst wird, um den Zugang mit dem Fräsgerät zu darunter liegenden Stellen zu ermöglichen. Damit wird nicht nur viel Material verbraucht, sondern damit ist auch ein hoher Zeitverbrauch verbunden.

### Zusammenfassung der Erfindung

Daher ist es ein Ziel der vorliegenden Erfindung, die vorgenannten Nachteile zumindest teilweise zu überwinden. Dazu wird gemäß eines Aspektes ein Verfahren zur extraoralen Herstellung eines Zahnersatzteils vorgestellt, das das Zur Verfügung Stellen eines Basiskörpers, das Drehschleifen, und das Schleifen des Basiskörpers umfasst.

Das vorgestellte Verfahren erlaubt ein besonders zeitgünstiges Verfahren zur Herstellung von Zahnersatzteilen. Die im Stand der Technik benötigte Zeit von bis zu 15min kann häufig halbiert werden, da ein hoher Materialabtrag in kurzer Zeit durch die Kombination aus Drehschleifen und Schleifen bewerkstelligt wird. Zudem kann je nach Wahl des Basiskörpers auch die Menge an eingesetztem Material pro herzustellende Zahnersatzteil reduziert werden. Dies gilt insbesondere für die noch näher beschriebenen Stäbe, die hierin synonym zu "Stangen" benutzt werden.

Gemäß einem weiteren Aspekt wird eine Vorrichtung zur extraoralen Herstellung eines Zahnersatzteiles zur Verfügung gestellt, wobei die Vorrichtung eine Einrichtung zum Drehschleifen sowie eine Einrichtung zum Schleifen umfasst.

Weitere vorteilhafte Ausgestaltungen, Weiterbildungen und Details der Erfindung werden aus den Unteransprüchen, der Beschreibung sowie den Figuren ersichtlich.

Das herzustellende Zahnersatzteil ist typsicher Weise ein individuell zu fertigendes Zahnersatzteil, insbesondere eine Einzelkrone und/oder eine mehrgliedrige Brücke. Das herzustellende Zahnersatzteil hat insbesondere keine für mehrere Zahnersatzteile identische Form. Das herzustellende Zahnersatzteil ist des Weiteren regelmäßig keine Einrichtung zur Verankerung im Kieferknochen wie zum Beispiel ein Implantat.

Der Ausdruck "Drehschleifen" wird hierin synonym zum "Drehen" benutzt. Es bezieht sich gemäß DIN 8580 auf die Hauptgruppe Trennen und die Gruppe Spanen mit geometrisch bestimmter Schneidenform. Nach DIN 8589 ist Drehen "Spanen mit geschlossener und meist kreisförmiger Schnittbewegung und beliebiger Vorschubbewegung in einer zur Schnittrichtung senkrechten Ebene. Die Drehachse der Schnittbewegung behält ihre Lage zum Werkstück unabhängig von der Vorschubbewegung bei." Das hierin dargestellte Drehschleifen umfasst insbesondere die Rotation des Basiskörpers, aus dem die Zahnersatzteile herzustellen sind.

Drehschleifen kann Plandrehen, Abstechdrehen, Runddrehen, Profildrehen, und/oder Formdrehen umfassen. Ausführungsformen der hierin beschriebenen Erfindung umfassen insbesondere das Formdrehen, demgemäß ggfs. computergesteuert auch Strukturen wie z. B. Vertiefungen und Kerben in dem Zahnersatzteil geformt werden. Dabei ist zu beachten, dass die Formgebung durch Drehschleifen typischer Weise rotationssymmetrisch erfolgt.

Schleifen ist ein spanendes Fertigungsverfahren zur Bearbeitung von Oberflächen mit Schleifmitteln. Nach DIN 8580 gehört es zur Hauptgruppe Trennen, und zwar zur Gruppe der Zerspanungsarten mit geometrisch unbestimmter Schneide. Beim Schleifen wird im Gegensatz zum Drehschleifen die Schneide in eine typischer Weise im Vergleich zum Basiskörper schnelle Bewegung gesetzt, wobei der Basiskörper dabei normaler Weise ruht.

Allerdings ist es auch möglich, dass sich dabei auch der Basiskörper mit einer typischer Weise geringen Drehzahl dreht, wie zum Beispiel mit einer Drehzahl zwischen 5 und 500 Umdrehungen pro Minute. Durch diese Kombination kann bei geeigneter Steuerung der Schleifschneide zum Beispiel der Basiskörper in eine ovale Form gebracht werden.

Gemäß hierin beschriebenen Ausführungsformen ist die Schleifschneide rotationssymmetrisch und wird in eine Rotationsbewegung gebracht. Es handelt sich insbesondere um Rundschleifen (Längs-Rundschleifen oder Quer-Rundschleifen). Die Schleifschneide ist gemäß Ausführungsformen ein Rundkörper mit einem Durchmesser von mindestens 2 cm, typischer Weise von mindestens 3 cm. Die aus Bindemittel und Körner bestehende Schleifschneide hat eine typische Aussiebungsgröße zwischen FEPA F220 bis FEPA F80.

Die Rotationsgeschwindigkeit beim Drehen ist typischer Weise zwischen 500 und 5000 Umdrehungen pro Minute, d.h., das Basisstück dreht sich in dieser Geschwindigkeit. Beim Schleifen ist die Rotationsgeschwindigkeit der Schleifoberfläche typischer Weise zwischen mindestens 500 Umdrehungen bzw. mindestens 2.000 Umdrehungen pro Minute. Dies ist regelmäßig abhängig vom Schleifscheibendurchmesser. So können beispielsweise bei einem Durchmesser von 15-25 mm Rotationsgeschwindigkeiten von 2.000 bis 30.000 Umdrehungen pro Minute vorkommen, und bei einem Durchmesser von 200-300 mm Rotationsgeschwindigkeiten von 500 bis 600 Umdrehungen pro Minute.

Gemäß hierin beschriebenen Ausführungsformen umfasst das Verfahren einen oder mehrere folgender zusätzlicher Schritte:
- Einspannen des Basiskörpers in eine Drehvorrichtung; und/oder
- Fräsen des Basiskörpers.

Durch das Drehschleifen kann schnell und effektiv ein großer Materialabtrag stattfinden. Die für den gleichen Abtrag vergleichbare Zeit im Falle von Fräsen wäre ein Vielfaches der Zeit, die beim Drehschleifen benötigt wird. Zudem ist der Schnittdruck beim Drehschleifen regelmäßig geringer als beim Fräsen, was für das Material und das herzustellende Zahnersatzteil vorteilhaft ist. Typischer Weise wird die Menge an per Drehen abzuschleifendem Materialabtrag pro herzustellendem Zahnersatzteil maximiert.

Gemäß Ausführungsformen findet zunächst ein Drehschleifen des Basiskörpers statt. Der Basiskörper ist typischer Weise rotationssymmetrisch und hat eine Rotationsachse (hierin "Achse" genannt), er ist insbesondere zylindrisch bzw. stabförmig. Ein solcher Stab kann zum Beispiel mit dem Stranggussverfahren hergestellt worden sein und kann eine Länge von zwischen 0.25 m und 2.0 m haben.

Als typischer Weise zweiter Schritt findet das Schleifen statt. Auch Schleifen erlaubt einen hohen Materialabtrag und einen dabei geringen Schnittdruck. Im Gegensatz zum Drehen ist Schleifen nicht beschränkt auf eine rotationssymmetrische Formgebung, sondern erlaubt einen lokalen Materialabtrag.

Das Fräsen des Basiskörpers zur Zahnersatzteilherstellung findet als Feinarbeitsschritt statt. Fräsen erfolgt lokal an den entsprechenden Stellen, während Drehschleifen zu einer rotationssymmetrischen Strukturgebung am Zahnersatzteil führt. Im Gegensatz zum Drehen wird die zur Spanabhebung notwendige Schnittbewegung beim Fräsen durch Rotation des Schneidewerkzeuges gegenüber dem fest im Maschinentisch eingespannten Basiskörper erzeugt. Im Gegensatz zum Schleifen handelt es sich bei dem Schneidwerkzeug jedoch um einen Rotationskörper kleiner Größe, wie zum Beispiel von maximal 10 mm, typischer Weise maximal 5 mm bzw. maximal 3 mm Durchmesser. Die zur Formgebung notwendige Vorschubbewegung kann je nach Bauart entweder durch Verschiebung des Maschinentisches oder durch Bewegung des Fräswerkzeuges relativ zum Basiskörper erreicht werden.

Das herzustellende Zahnersatzteil muss selbstverständlich derart bemessen sein, dass es aus dem gegebenen Durchmesser des Basiskörpers herstellbar ist. Durch das Drehschleifen wird der Basiskörper bis auf eine Größe reduziert, die dem maximalen Durchmesser des herzustellenden Zahnersatzteil entspricht. Typischer Weise beinhaltet dieses Drehschleifen Plandrehen und/oder Runddrehen.

Es ist weiterhin möglich, dass nach dem Fräsen ein Drehschleifen oder Schleifen folgt. Beispielsweise könnte zuerst in einem Drehschleif- und Schleifvorgang schnell und effizient ein hoher Materialabtrag stattfinden, wonach die genaue Fräsung der erwünschten Struktur erfolgt. Hieran anschließend könnte wieder ein Drehschleifen folgen, zum Beispiel ein Abstechdrehen oder ein Drehen bis zum nahezu Abstechen, bspw. um das dann fertig gestellte Zahnersatzteil vom Basiskörper zu lösen. Auch dieses Ablösen durch Abstechdrehen geht schnell und ist dem Fräsen meist überlegen. Gemäß weiteren Ausführungsformen findet pro Zahnersatzteil eine mehrfache Abfolge von Drehen und Fräsen statt.

Gemäß einer beispielhaften Ausführungsform wird pro herzustellendem Zahnersatzteil der Basiskörper zunächst bspw. durch Runddrehen oder Plandrehen auf den maximalen Durchmesser des herzustellenden Zahnersatzteils abgespant. Weiterer Materialabtrag erfolgt mittels Schleifen. Zur feinen Strukturgebung kann schließlich in einem weiteren Schritt Fräsen erfolgen.

Das Verfahren kann des Weiteren das Befestigen des Basiskörpers auf einem Drehteller umfassen. Der Basiskörper kann zum Beispiel in eine auf dem Drehteller fest verbundene Einspanneinrichtung eingespannt werden. Der Drehteller in der hierin verstandenen Bedeutung ist eine Unterlage, die konfiguriert ist, sich und den Basiskörper mit einer ggfs. vorgebbaren Geschwindigkeit zu drehen. Darüber hinaus kann der Drehteller typischer Weise fixiert werden, um bspw. Schleifen und/oder Fräsen an einem positionsfesten Körper vornehmen zu können.

Gemäß einer typischen Ausführungsform ist der Basiskörper rotationssymmetrisch, insbesondere zylinderförmig. Der Basiskörper ist beispielsweise ein Stab, und kann eine Länge haben von mindestens 10 cm, typischer Weise mindestens 20 cm bzw. mindestens 50 cm. Je länger der Stab ist, desto mehr Zahnersatzteil können aus dem Stab gefertigt werden. Gleichzeitig reduziert sich der Aufwand zum Befestigen des Stabes auf dem Drehteller.

Gemäß hierin beschriebenen Ausführungsformen besteht der Basiskörper aus einer Cobalt-Chrom-Legierung, insbesondere einer Chrom-Cobalt-Molybdän-Legierung. Je nach Hersteller enthält sie in etwa 62-66 % Cobalt, 27-31 % Chrom, 4-5 % Molybdän sowie Spuren von Kohlenstoff, Silizium, Mangan, Eisen, und/oder anderen Bestandteilen. Besonders bevorzugt wird ein Stab aus Keragen®, der insbesondere durch ein Stranggussverfahren hergestellt wurde und daher frei von Lunkern und Gasblasen ist. Keragen® ist eine Cobalt-Chrom-Legierung, die kein Molybdän enthält. Sie hat sich als haltbares und gut verträgliches Material erwiesen. Typische Durchmesser liegen zwischen 7 mm und 20 mm, insbesondere zwischen 8 mm und 12 mm wie bei 8 mm, 9,5 mm oder 12 mm. Sie sind typischer Weise durch ein Strangguss, ein Glassaugguss oder durch einen gesinterten HIP (Hot Isostatic Pressuring) Prozess hergestellt worden. Sie weisen damit eine hohe Homogenität auf, und sind frei oder nahezu frei von Lunkern und Gasblasen.

Auf Grund der hohen Schmelztemperatur von mindestens 1.200°C werden Zahnersatzteile aus der Cobalt-Chrom-Legierung häufig durch spanende Verfahren hergestellt. Die Cobalt-ChromLegierung hat sich in der zahnärztlichen Praxis bewährt, da sie eine hohe Härte aufweist, völlig korrosionsfrei ist, und zu keinen Verfärbungen im Munde durch Einflüsse wie Speichel oder Nahrung führt. Die Rate an allergischen Reaktionen bei Patienten ist gering, ihre Verträglichkeit ist hoch. In manchen Krankensystemen ist zudem vorteilhaft, dass die Kosten für den Zahnersatz aus dieser Legierung von der Krankenkasse übernommen werden, während die Kosten für Biomaterialien wie Gold, Platin, oder Titan nicht übernommen werden.

Nach Ausführungsformen wird das Verfahren teilweise oder vollständig von einem Computer ausgeführt. Dabei steuert ein Computer das erfindungsgemäße Drehen und/oder Schleifen und ggfs. auch weitere Vorgänge wie zum Beispiel Fräsen. Der Computer kann insbesondere an ein Netz angeschlossen sein, wie zum Beispiel dem world wide web, worüber ihm Daten aufgespielt werden, wie die gewünschten Zahnersatzteile auszusehen haben. Beispielsweise können derartige Daten in Form von einer STL-Datei auf den Computer übertragen werden. Der Computer kann eine Eingabeeinheit, wie z. B. eine Maus und/oder eine Tastatur, eine Darstellungseinheit, wie z. B. einen Bildschirm, eine Recheneinheit, wie z. B. eine CPU (central processing unit), und eine Speichereinheit, wie z. B. einen Permanentspeicher, bspw. eine Festplatte, und/oder einen flüchtigen Speicher, wie z. B. einen RAM (Random Access Memory), aufweisen. Der Computer steuert die Drehschleifmaschine, die gemäß der hierin beschriebener und mit allen anderen hierin beschriebenen Ausführungsformen kombinierbarer Ausführungsform mindestens einen Drehmeißel, einen Drehteller sowie Befestigungsmittel für die einzuspannenden Stangen aufweist. Der Computer kann auch das Fräßwerkzeug steuern, mit dessen Hilfe Feinarbeit an dem herzustellenden Zahnersatzteil vorgenommen wird.

Typischer Weise findet das erfindungsgemäße Verfahren teil- oder vollautomatisch statt. Dabei kann die benötigte Arbeitszeit eines Bearbeiters, wie z.B. eines Zahntechnikers, reduziert werden und dabei Kosten gespart werden.

Nach Ausführungsformen umfasst das Verfahren auch Abstechen des Zahnersatzteils vom Basiskörper. Das Abstechen findet typischer Weise nach Fertigstellung der gewünschten Struktur auf dem Zahnersatzteil statt.

Typischer Weise wird jedes herzustellende Zahnersatzteil individuell gefertigt. Bei dem vorgestellten Verfahren handelt es sich somit nicht um die Massenproduktion eines immer gleich strukturierten Werkstückes der Zahnheilkunde, sondern um die nach individuellen Vorgaben hergestellte Form eines Zahnersatzteils. Mitunter aus diesem Grund wurde es im Stand der Technik bisher nicht in Erwägung gezogen, eine Kombination aus Drehschleifen und Schleifen zur Herstellung von Zahnersatzteilen zur Verfügung zu stellen.

### Kurze Beschreibung der Figuren

Nachfolgend wird die Erfindung beispielhaft anhand der begleitenden Figuren erläutert.
Fig. 1 zeigt eine Basisplatte zur Zahnersatzteilherstellung aus dem Stand der Technik vor der Bearbeitung.
Fig. 2 zeigt eine Basisplatte zur Zahnersatzteilherstellung aus dem Stand der Technik nachdem der Zahnersatz herausgefräst wurde.
Fig. 3 zeigt beispielhaft mögliche Ausführungsformen eines Basiskörpers zur Verwendung in dem erfindungsgemäßen Verfahren.
Figuren 4 und 5 zeigen schematisch mögliche Ausführungsformen einer erfindungsgemäßen Vorrichtung.
Figuren 5-8 zeigen schematisch eine mögliche Ausführungsform einer virtuellen Einbettung gemäß eines optionalen erfindungsgemäßen Schrittes.
Fig. 9 illustriert beispielhaft den Materialabtrag mittel Drehen, Schleifen und Fräsen gemäß einer Ausführungsform.
Fig. 10 zeigt mögliche Ausführungsformen des erfindungsgemäßen Verfahrens.
Fig. 11 zeigt schematisch eine mögliche Ausführungsform einer erfindungsgemäßen Vorrichtung.

### Ausführliche Beschreibung der Ausführungsformen

In den folgenden Figuren bezeichnet die gleiche Referenznummer das gleiche Element.

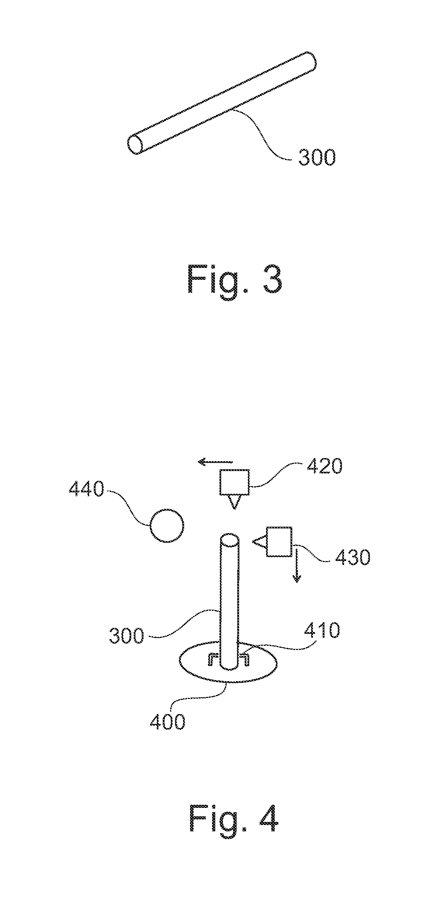

Fig. 3 zeigt beispielhaft eine mögliche Ausführungsform eines Basiskörpers zur Verwendung in dem erfindungsgemäßen Verfahren. Der stabförmige Basiskörper, der zum Beispiel aus einer Chrom-Cobalt-Legierung besteht, wird erfindungsgemäß zur Herstellung eines Zahnersatzteil mittels Drehen benutzt. Eine typische Länge des stabförmigen Basiskörpers beträgt bis zu 2 m.

Dabei funktioniert das Verfahren gemäß der in Fig. 4 veranschaulichten Ausführungsform wie folgt: Der Basiskörper 300 wird bspw. mittels einer Einspanneinrichtung 410 auf dem Drehteller 400 verankert. Der Drehteller kann durch einen Motor betrieben werden. Die Rotationsdrehzahl sollte mit der idealen Schnittgeschwindigkeit abgestimmt sein.

Beispielhaft und schematisch sind in der Ausführungsform von Fig. 4 des Weiteren die Schleifmeißel 420 und 430 gezeigt. Gemäß einer anderen Ausführungsform wird lediglich ein Schleifmeißel eingesetzt, der optional positions- und richtungsverstellbar ist. Die Darstellung von Fig. 4 kann als Darstellung zweier, unterschiedlich gerichteter Schleifmeißel angesehen werden, oder aber als Darstellung lediglich eines Schleifmeißel zu zwei verschiedenen Zeitpunkten.

Gemäß hierin beschriebenen Ausführungsformen kann das Verfahren ein Plandrehen umfassen. Dies ist auch in der Fig. 4 mit dem Drehmeißel 420 angedeutet. Beim Plandrehen ist der Schleifmeißel in Richtung der Achse des Basiskörpers ausgerichtet, wobei die Richtung der Bewegung des Schleifmeißels ("Vorschubrichtung") in radialer Richtung bezogen auf die Achse des Basiskörpers erfolgt. Dies ist durch den über dem Drehmeißel 420 eingezeichneten Pfeil angedeutet.

Das Verfahren kann auch ein Runddrehen umfassen. Dies ist auch in der Fig. 4 mit dem Drehmeißel 430 angedeutet. Beim Runddrehen ist der Schleifmeißel quer zur Richtung der Achse des Basiskörpers ausgerichtet, das heißt also in seiner radialen Richtung, wobei die Vorschubrichtung in axialer Richtung bezogen auf die Achse des Basiskörpers erfolgt. Dies ist ebenfalls durch einen Pfeil neben dem Schleifmeißel 430 angedeutet.

Insbesondere bei der wiederholten Herstellung ähnlicher Morphologien ist es auch denkbar, dass ein Profildrehen zum Einsatz kommt. Beim Profildrehen wird bereits eine Textur im Schleifkörper nachgebildet, so dass pro Zeitpunkt nicht nur ein Kontaktpunkt zwischen Schleifmeißel und herzustellendem Zahnersatzteil vorliegt, sondern eine möglicher Weise einige mm überstreichende Fläche. Dies erniedrigt zusätzlich die benötigte Herstellungszeit für ein Zahnersatzteil.

Darüber hinaus zeigt die Ausführungsform von Fig. 4 auch eine Schleifscheibe 440. Die Achse der Schleifscheibe ist vorliegend senkrecht zur Achse des Basiskörpers dargestellt. Gemäß Ausführungsformen ist die Achse der Schleifscheibe koaxial zur Achse des Basiskörpers angeordnet.

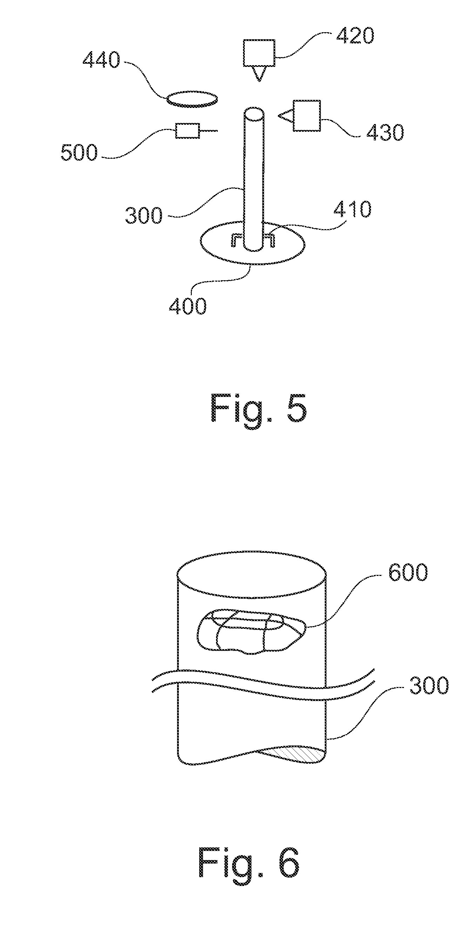

Fig. 5 veranschaulicht weitere Ausführungsformen. Neben dem zum Drehschleifen genutzten Drehmeißel 420 umfasst die gezeigte Anordnung auch einen Fräßkopf 500, der zum Fräsen eingesetzt wird. Wie bereits beschrieben, erfolgt nach hierin beschriebenen Ausführungsformen auch ein Fräßvorgang. Darüber hinaus ist die Schleifscheibe 440 zum Schleifen mit zum Basiskörper koaxialer Ausrichtung dargestellt. Typischer Weise findet der Fräßvorgang nach dem Schleifvorgang statt, der wiederum häufig nach dem Drehschleifvorgang statt findet.

Typischer Weise findet das erfindungsgemäße Verfahren teil- oder vollautomatisch statt. Die folgenden, in Bezug zu Figuren 6-8 beschriebenen Schritte können entweder von einem Betreuer wie einem Zahntechniker vorgenommen werden, oder sie werden als Softwareschritte von dem Computer übernommen.

Gemäß Ausführungsformen werden sämtliche herzustellende Zahnersatzteil zunächst einmal in virtuelle Gruppen aufgeteilt, wobei alle Gruppenmitglieder zu ihrer Herstellung einen ähnlichen maximalen Stabdurchmesser benötigen. So kann es beispielsweise die Gruppe der kleinen Zahnersatzteils geben, die mit einem Stabdurchmesser von ca. 8 mm hergestellt werden können, die Gruppe der mittelgroßen Zahnersatzteile, die mit einem Stabdurchmesser von ca. 9,5 mm hergestellt werden können, und die Gruppe der großen Zahnersatzteile, die die mit einem Stabdurchmesser von ca. 12 mm hergestellt werden können. Typische Anzahl von verschiedenen Gruppen sind 2, 3, 4 oder sogar 5. Dies hängt auch wesentlich von der Anzahl der innerhalb einer vorgegebenen Zeit herzustellenden Zahnersatzteile ab. Ziel dieser Aufteilung ist es, die Menge an abzuspanendem Material so stark wie möglich zu reduzieren.

Mit beispielhaftem Bezug zu Fig. 6 wird das herzustellende Zahnersatzteil, das in den Figuren mit 600 bezeichnet ist, virtuell in den Basiskörper 300 gelegt. Dabei wird gemäß Ausführungsformen, die mit anderen hierin beschriebenen Ausführungsformen kombiniert werden können, das herzustellende Zahnersatzteil so in den Basiskörper gelegt, dass der benötigte Materialeinsatz minimiert wird. Zudem kann die Optimierung so stattfinden, dass möglichst viel von dem abzuspanendem Material mit Drehschleifen abgespant wird. In Fällen, wo es einen zusätzlichen Fräsvorgang gibt, kann die Optimierung auch derart sein, dass möglichst wenig von dem abzuspanendem Material mit Fräsen abgespant wird.

Eine weitere mögliche Optimierung in dem erfindungsgemäßen Verfahren betrifft die Maximierung der Anzahl der herzustellenden Zahnersatzteile aus einem Basiskörper. Beispielsweise werden die herzustellenden Zahnersatzteile derart in den Basiskörper gelegt, dass jeweils die von ihnen verbrauchte Höhe minimiert wird.

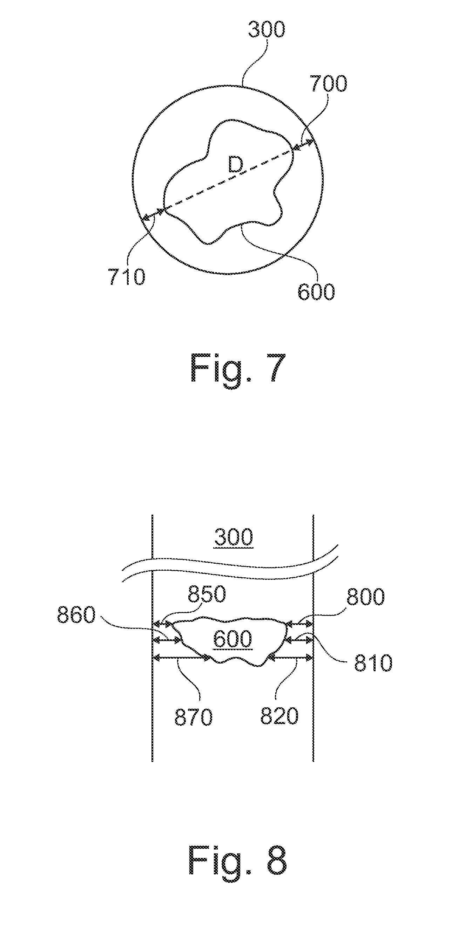

Bei einer Ausführungsform des Verfahrens wird aus den aufgespielten Daten das herzustellende Zahnersatzteil virtuell in den vorhandenen Basiskörper gelegt. Dies ist in einer dreidimensionalen Ansicht in Fig. 6 veranschaulicht. Fig. 7 zeigt den hierzu passenden Querschnitt. Man erkennt, dass das herzustellende Zahnersatzteil 600 derart in dem Basiskörper 300 positioniert wird, dass der Abstand 700 und 710 zum Rand verbleibt. Bei einer rein zweidimensionalen Optimierung wäre es im Sinne einer Optimierung der mit Drehen abzuspanenden Materialmenge, dass der Abstand 700 und 710 gleich groß ist, denn der kleinste Abstand zum Rand hin bestimmt, bis auf welchen Durchmesser D das Zahnersatzteil durch Drehschleifen abgespant werden kann.

Gemäß einer Ausführungsform der vorliegenden Offenbarung wird eine dreidimensionale Optimierung durchgeführt. Der Basiskörper wird beispielsweise auf unterschiedlicher axialer Höhe in unterschiedlich starkem Ausmaß geschliffen. Dies ist in Fig. 8 veranschaulicht, die einen Längsschnitt durch den Basiskörper samt virtuell eingefasstem Zahnersatzteil darstellt. Das schematisch dargestellte Zahnersatzteil 600 soll im oberen Bereich eine größere Ausdehnung haben als im weiter unten liegenden Bereich (wobei die Bezeichnung mit "oben" und "unten" genauso wie die noch folgende Bezeichnung "links" und "rechts" lediglich den Bezug zu der Figur erleichtern soll). Durch Drehen, insbesondere Plandrehen oder Runddrehen, kann für unterschiedliche axiale Höhen ein unterschiedlicher Durchmesser des Basiskörpers abgetragen werden. So zeigt Fig. 8 im oberen Bereich einen Abstand 850 zum linken Rand sowie einen Abstand 800 zum rechten Rand. Auf Grund der Gesamtoptimierung mag es sein, dass der Abstand 800 nicht dem Abstand 850 entspricht. Auf dieser Höhe darf also lediglich bis zum kleineren Abstand der Abstände 800 und 850 drehgeschliffen werden.

Bei der für die Ausführungsform von Fig. 8 gezeigten Darstellung des Zahnersatzteil wird deutlich, dass das Drehen in weiter unten liegenden Höhen nicht nur bis zum kleineren Abstand von den Abständen 800 und 850 reichen sollte, sondern auf Grund der gewünschten Form ein größerer Materialabtrag durch das schnelle und materialschonende Drehschleifen erfolgen sollte. Also wird in der gezeigten mittleren Höhe bis auf den kleineren Abstand der Abstände 810 und 860 drehgeschliffen, und in der gezeigten unteren Höhe bis auf den kleineren Abstand der Abstände 820 und 870 drehgeschliffen. Allgemein ausgedrückt heißt das, dass gemäß Ausführungsformen der Basiskörper je nach Höhe bis auf unterschiedliche Durchmesser hin durch Drehschleifen abgespant wird. Typischer Weise erfolgt danach noch ein Schleifschritt, gemäß Ausführungsformen werden weitere Materialabträge durch Fräsen vorgenommen.

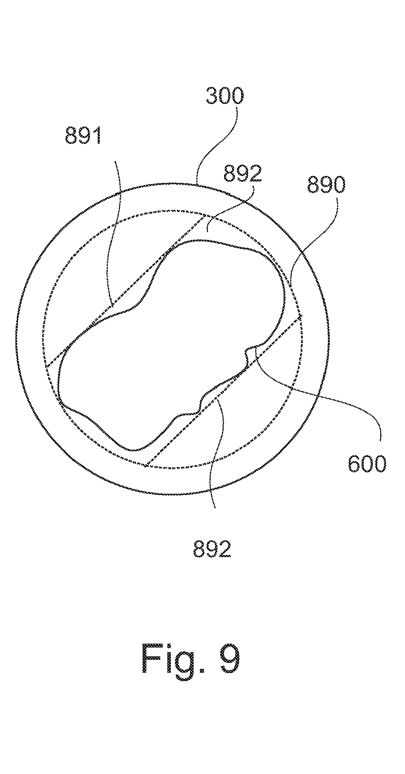

Fig. 9 veranschaulicht den Materialabtrag durch unterschiedliche Verfahren. Herzustellen ist das Zahnersatzteil 600 mit dem gezeigten Querschnitt. Bis zum gestrichelt gezeichneten Kreis 890 wird der Materialabtrag aus dem Basiskörper 300 mit Hilfe von Drehschleifen vorgenommen. Damit wird ein umfangreicher Materialabtrag in einer zeiteffizienten Weise vorgenommen. Den weiteren größeren Materialabtrag, der nicht mehr rotationssymmetrisch vorgenommen werden kann, erfolgt nun per Schleifen bis zu den gezeichneten Linien 891 auf der einen Seite und 892 auf der anderen Seite. Dies kann bspw. durch Schleifen am ruhenden Basiskörper vorgenommen werden. Es ist auch möglich, dass zumindest ein Teil des Materialabtrags durch Schleifen an einem drehenden Basiskörper stattfindet. Zum Beispiel, durch einen variierenden Andruck der Schleifscheibe bei einem sich langsam (d.h., bspw. mit weniger als 500 Umdrehungen pro Minute) drehenden Basiskörper kann gezielt eine ovale Form hergestellt werden.

In der gezeigten Ausführungsform von Fig. 9 wird der Rest mittels Fräsen entfernt. In anderen Ausführungsformen könnten noch weitere Teilbereiche mittels Schleifen entfernt werden, z. B. ein Großteil des mit 892 bezeichneten Bereichs. Feinere Konture, insbesondere Kerben und Vertiefungen können regelmäßig jedoch nur mit Fräsen hergestellt werden.

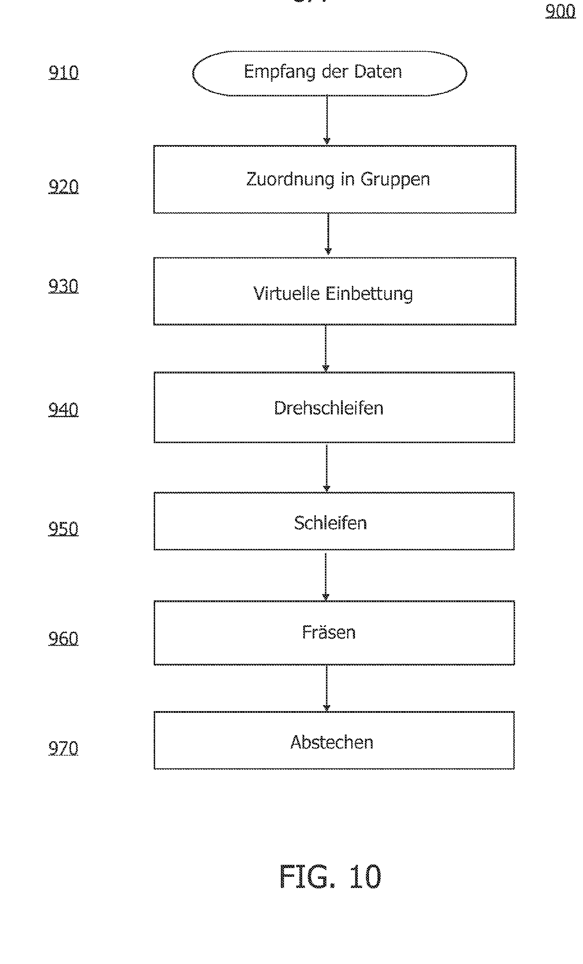

Fig. 10 veranschaulicht ein typisches Vorgehen nach einer Ausführungsform des erfindungsgemäßen Verfahren. Dabei stellt dies lediglich ein Beispiel dar und darf nicht beschränkend ausgelegt werden. Einige der einzelnen gezeigten Schritte können ausgelassen werden bzw. alternativ gelöst werden.

Das beispielhaft dargestellte Verfahren 900 beginnt mit dem Empfang der Daten in Block 910. Diese umfassen in der Regel mindestens eine Datei, wie zum Beispiel eine STL-Datei, normaler Weise mehrere Dateien über herzustellende Zahnersatzteile.

Gemäß den empfangenen Daten werden die herzustellenden Zahnersatzteile in Block 920 optional in virtuelle Gruppen eingeteilt. Wie bereits dargelegt, können verschiedene Gruppen gebildet werden, die unterschiedlich große Basiskörper benötigen, um sie mit Basisstangen unterschiedlich großen Durchmessers herzustellen. Typische Durchmesser liegen bei mindestens 8 mm, häufig bis 12,5 mm. Zum Beispiel könnte eine erste Gruppe für herzustellende Zahnersatzteile mit maximalem Durchmesser von 8 mm gebildet werden, eine zweite Gruppe für herzustellende Zahnersatzteile mit maximalem Durchmesser von 9,5 mm gebildet werden, und eine dritte Gruppe für herzustellende Zahnersatzteile mit maximalem Durchmesser von 12 mm gebildet werden. Der optionale Schritt der Gruppeneinteilung erlaubt zusätzlich die Materialoptimierung.

Als nächster Block 930 werden die herzustellenden Zahnersatzteile virtuell in die gewählten Basiskörper eingebettet. Diese Einbettung kann manuell oder von einem Computer erfolgen. Die bereits genannten Optimierungsbedingungen werden normaler Weise befolgt.

Gemäß der virtuellen Einbettung wird schließlich das Zahnersatzteil hergestellt. Gemäß Block 940 wird in einem ersten Schritt zunächst durch Drehen ein rotationssymmetrischer Materialabtrag durchgeführt. Dies kann für jede axiale Höhe des Basiskörpers bis zu einem eigenen Durchmesser erfolgen. Die Vorgabe dieses Schrittes ist es regelmäßig, einen möglichst hohen Materialabtrag durch Drehen zu erreichen.

Gemäß Block 950 folgt in einem weiteren Schritt ein Schleifen des Basiskörpers. Dabei ist es Ziel, einen Großteil des nicht-rotationssymmetrischen Materialabtrags vorzunehmen.

In Block 960 folgt ein Ausfräsen von weiteren Spanen. Das Fräsen kann manuell oder computergesteuert erfolgen. Das Fräsen erlaubt die genaue lokale Strukturgebung.

Grundsätzlich kann sich Drehen, Schleifen und Fräsen beim identischen Zahnersatzteil abwechseln bzw. die einzelnen Schritte wiederholen. Darüber hinaus ist es auch denkbar, Schleifen und Fräsen an dem Formkörper parallel vorzunehmen.

Nach weitestgehender Fertigstellung der gewünschten Struktur des Zahnersatzteils wird dieses vom Basiskörper gelöst. Dies kann durch Abstechdrehen oder durch Fräsen erfolgen, und ist in Fig. 9 durch den Block 960 angezeigt. Es ist möglich, dass zusätzlich eine Zangeneinheit (nicht gezeigt) während des Abstechens das Zahnersatzteil greift. Die Zangeneinheit, die manuell oder computergesteuert sein kann, kann auch genutzt werden, um das Zahnersatzteil direkt dorthin zu befördern, wo es entweder gelagert wird oder schließlich zur weiteren Verwendung gebraucht wird.

Je nach weiter noch herzustellenden Zahnersatzteilen, werden die Schritte wiederholt. Zum Beispiel kann direkt mit Block 930, der virtuellen Einbettung des als nächstes herzustellendem Zahnersatzteil fortgefahren werden. Der Basiskörper muss typischer Weise nach einer Zahnersatzteilherstellung nicht ausgetauscht werden sondern eignet sich für die Herstellung von einigen, normaler Weise mindestens 10 Zahnersatzteilen.

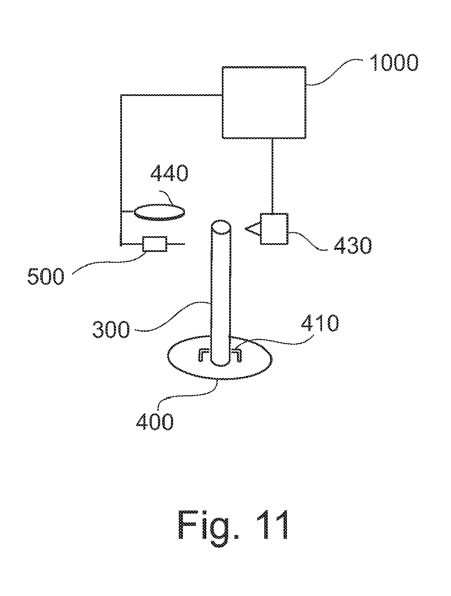

Fig. 11 zeigt schließlich eine Ausführungsform, wobei zur Illustration ein Computer 1000 gezeigt ist. Der Computer dient regelmäßig der Steuerung der Einrichtung zum Drehschleifen, in Fig. 11 schematisch dem gezeigten Drehmeißel 430, und der Einrichtung zum Schleifen, in

Fig. 11 der schematisch gezeigten Schleifscheibe 440. Darüber hinaus kann der Computer auch der Steuerung der Einrichtung zum Fräsen dienen, in Fig. 11 dem gezeigten Fräswerkzeug 500. Gemäß Ausführungsformen kann der Computer auch den Drehteller, der in Fig. 11 schematisch als 400 gezeigt ist, dienen. Der Computer ist typischer Weise mit dem Internet verbunden.

Das vorliegende Verfahren und die vorgestellte Vorrichtung ist zur Herstellung von Zahnersatzteilen ausgerichtet. Damit müssen die Abspangeräte (Drehmeißel, Schleifschneide, Fräsgerät) mit Materialien höchster Härte zurechtkommen (Brinellhärte von bis zu 400 kg/mm² bzw. nach DIN EN ISO 6506-1 Stand: 03/2006: 400 HBW 10/3000), insbesondere mit Chrom-Cobalt-Legierungen. Ebenso elementar ist die Eignung der Abspangeräte, auf eine Genauigkeit von 5 µm oder sogar 3 µm Strukturen erzeugen zu können.

## Patentansprüche

1. Verfahren zur extraoralen Herstellung eines Zahnersatzteiles, umfassend:
- Zur Verfügung Stellen eines Basiskörpers (300);
- Drehschleifen des Basiskörpers; und
- Schleifen des Basiskörpers.

2. Verfahren nach einem der vorhergehenden Ansprüche, wobei der Basiskörper rotationssymmetrisch, insbesondere stabförmig ist.

3. Verfahren nach Anspruch 2, wobei der stabförmige Basiskörper einen Durchmesser von wenigstens 8 mm, optional von wenigstens 9,5 mm aufweist.

4. Verfahren nach einem der vorhergehenden Ansprüche, des Weiteren umfassend:
- Fräsen des Basiskörpers.

5. Verfahren nach einem der vorhergehenden Ansprüche, wobei der Basiskörper aus einer Chrom-Cobalt-Legierung besteht.

6. Verfahren nach einem der vorhergehenden Ansprüche, wobei das Verfahren gemäß folgender Reihenfolge ausgeführt wird:
- Drehschleifen des Basiskörpers;
- Schleifen des Basiskörpers; und
- optionales Fräsen des Basiskörpers.

7. Verfahren nach einem der vorhergehenden Ansprüche, des Weiteren einen oder mehrere folgender Vorgänge umfassend:
- Empfangen von Daten zur Herstellung des Zahnersatzteils;
- Einspannen des Basiskörpers auf einem Drehteller; und
- Abdrehen des fertig gestellten Zahnersatzteils.

8. Verfahren nach einem der vorhergehenden Ansprüche, wobei das Drehschleifen, das Schleifen, und optional das Fräsen mit einer Genauigkeit von 5 µm, optional 3 µm erfolgt.

9. Verfahren nach einem der vorhergehenden Ansprüche, wobei das Schleifen des Basiskörpers vorgenommen wird, während sich der Basiskörper dreht.

10. Computerprogrammprodukt zur Ausführung auf einer Datenverarbeitungsanlage, das ausführbare Befehle für das Verfahren nach einem der vorhergehenden Ansprüche umfasst.

11. Vorrichtung zur extraoralen Herstellung eines Zahnersatzteiles aus einem Basiskörper (300), umfassend:
- eine Einrichtung zum Drehschleifen (420, 430) des Basiskörpers (300); und
- eine Einrichtung zum Schleifen (440) des Basiskörpers.

12. Vorrichtung nach Anspruch 11, wobei der Basiskörper rotationssymmetrisch, insbesondere stabförmig ist, und einen Durchmesser von wenigstens 8 mm, optional von wenigstens 9,5 mm aufweist, und optional aus einer Chrom-Cobalt-Legierung besteht.

13. Vorrichtung nach einem der Ansprüche 11-12, des Weiteren umfassend:
- eine Einrichtung zum Fräsen (500) des Basiskörpers.

14. Vorrichtung nach einem der Ansprüche 12-13, des Weiteren einen oder mehrere folgender Elemente umfassend:
- einen Computer (1000) zum Empfangen von Daten zur Herstellung des Zahnersatzteils und/oder Steuern zumindest einer der Einrichtung zum Drehschleifen, der Einrichtung zum Schleifen, und der Einrichtung zum Fräsen des Basiskörpers; und
- eine Einspanneinrichtung (410) zur Fixierung des Basiskörpers auf einem Drehteller.

15. Vorrichtung nach einem der Ansprüche 11-14, wobei die Einrichtung zum Drehschleifen, die Einrichtung zum Schleifen, und/oder die Einrichtung zum Fräsen eingerichtet ist, mit einer Genauigkeit von mindestens 5 µm, optional 3 µm zu spanen.
